# EUROPEAN PATENT SPECIFICATION

(11) **EP 3 624 772 B1**
(45) Date of publication and mention of the grant of the patent: **27.10.2021**
(21) Application number: 18730451.4
(22) Date of filing: 17.05.2018
(51) Int. Cl.: A61K 9/00, A61K 9/06, A61K 36/185, A61K 31/205, A61K 31/355, A61K 36/28, A61K 36/38, A61K 36/63, A61K 36/886, A61K 47/10, A61K 47/44

(54) **COMPOSITION FOR THE TREATMENT OF FORMS OF SKIN INFLAMMATION**
ZUSAMMENSETZUNG ZUR BEHANDLUNG VON HAUTENTZÜNDUNGSFORMEN
COMPOSITION POUR LE TRAITEMENT DE FORMES D'INFLAMMATION CUTANÉE

(30) Priority: 19.05.2017 IT 201700054488
(43) Date of publication of application: 25.03.2020
(73) Proprietor: Muscas, Giancarlo, 09037 San Gavino Monreale (IT)
(72) Inventor: Muscas, Giancarlo, 09037 San Gavino Monreale (IT)
(74) Representative: Modiano, Micaela Nadia
(86) International application number: PCT/IB2018/053463
(87) International publication number: WO 2018/211445

(56) References cited:
- WO-A1-01/82878
- US-A1- 2011 280 969
- US-B1- 8 846 114
- HAVVA OZGE KESEROGLU ET AL: "Prevalence of herbal therapy usage in patients with psoriasis in Turkey", TANG [HUMANITAS MEDICINE], vol. 5, no. 2, 31 May 2015 (2015-05-31), pages 13.1-13.5, XP055441033, DOI: 10.5667/tang.2014.0029
- UTE WÖLFLE ET AL: "Topical Application of St. Johnʼs Wort (Hypericum perforatum)", PLANTA MEDICA, vol. 80, no. 02/03, 8 November 2013 (2013-11-08), pages 109-120, XP055440938, DE ISSN: 0032-0943, DOI: 10.1055/s-0033-1351019
- SALA A ET AL: "ANTI-INFLAMMATORY AND ANTIOXIDANT PROPERTIES OF HELICHRYSUM ITALICUM", JOURNAL OF PHARMACY AND PHARMACO, JOHN WILEY & SONS LTD, LONDON; GB, vol. 54, no. 3, March 2002 (2002-03), pages 365-371, XP008025203, ISSN: 0022-3573, DOI: 10.1211/0022357021778600
- Hobein ET AL: "Sweet Almond Oil with essential fatty acids", , 2012, pages 1-21, XP055441186, Retrieved from the Internet: URL:https://www.moja-lekarna.com/images/do cument/EUBOS%20HAUT%20RUHE%202.pdf [retrieved on 2018-01-16]

## Description

The present invention relates to a composition for the treatment of forms of skin inflammation.

Specifically, an application of such composition is in the topical treatment of states of skin inflammation, which can also be associated with thickening and flaking, such as for example psoriasis, lichen, dermatitis and erythema.

As is known, very often the topical treatment of skin conditions, associated with widely different diseases, passes through states of skin inflammation that specifically indicate the physiological response to an injury or occasionally to an immune hyperactivity, or even an immune reaction against endogenous agents recognized as non-self, as happens in psoriasis.

In psoriasis, which is taken here for the purposes of example as a skin inflammation state, the affected areas exhibit an excessively rapid development of keratinocytes that cannot conclude their maturation and differentiation pattern and form the mature horny layer of the skin that provides its barrier effect. Hence psoriasis manifests with well-defined red papules or erythematous plaques, covered with whitish or silvery dry flakes, sometimes associated with itching and burning sensations.

Psoriatic lesions are typically found at the elbows, knees, scalp and in the umbilical and sacral areas, and in histological terms they present as areas of hyper-proliferation, with an accelerated epidermic turnover approximately 10 times faster than normal skin, with incomplete maturation of keratinocytes and maintenance of nuclei in the horny layer.

Local treatment of psoriasis, as with other forms of skin inflammation, typically entails the local application of topical preparations containing softening substances such as Vaseline, or based on anti-inflammatory substances such as cortisone-based compounds, reducing agents such as tar and dithranol, or keratolytic agents such as salicylic acid and/or urea or, also, analogs of vitamin D.

However, the therapies used nowadays do not appear to offer satisfactory results in the complete remission of symptoms of diseases with inflammation of the skin.

In fact, the functionality of a topical formulation depends on its complete formula and not only on the type and concentration of active ingredients that it carries. Very often the topical treatment of skin conditions is limited to acting to combat and block the inflammation process, thanks to the presence of anti-inflammatory medicines, but it is not capable of restoring physiological skin conditions.

The topical formulations known today are not in fact capable of improving the hydration conditions and of promoting the permeation of the active ingredients through the horny layer, the principal skin barrier.

US 8,846,114 B1 discloses a topical composition with anti-inflammatory properties comprising olive oil, St John's wort oil, panthenol, water, *Aloe* and glycerol.

The aim of the present invention is to provide a composition for the treatment of forms of skin inflammation which is capable of improving the known art in one or more of the above mentioned aspects.

Within this aim, an object of the invention is to provide a composition for the treatment of forms of skin inflammation that is capable not only of reducing states of inflammation, but also of creating the hydration conditions that make it possible to break through the horny layer, thus enabling the active ingredients to act in the sites where they can actually carry out their action.

Another object of the invention is to provide a composition that is capable of rendering, in the body area where the component is applied, the environment protected from bacterial proliferation and from the action of free radicals.

Another object of the invention is to provide a composition that is capable of acting as a soothing and/or astringent agent in the area treated, thus reducing the typical symptoms associated with a state of inflammation.

Moreover, another object of the present invention is to overcome the drawbacks of the known art in an alternative manner to any existing solutions.

Another object of the invention is to provide a composition that is highly reliable, easy to implement and low cost.

This aim and these and other objects which will become better apparent hereinafter are achieved by a composition for the treatment of forms of skin inflammation, according to claim 1, optionally provided with one or more of the characteristics of the dependent claims.

The composition for the treatment of forms of skin inflammation, according to the invention, comprises at least the following components:
- olive oil,
- St John's wort oil,
- wax,
- cetostearyl alcohol,
- cetomacrogol,
- panthenol,
- tocopheryl acetate,
- water,
- freeze-dried *Aloe* extract,
- acetyl carnitine,
- *Helichrysum*,
- glycerol,
- a Geogard^{®} preservative,
- sodium ascorbate.

The same composition can also comprise beet extract and, preferably, it comprises the following components referred to 100 and in the following percentages:
- 13% olive oil,
- 3% St John's wort oil,
- 1% wax,
- 4% cetostearyl alcohol,
- 1.5% cetomacrogol,
- 0.5% panthenol,
- 1% tocopheryl acetate,
- 68.79% water,
- 1% freeze-dried *Aloe* extract,
- 0.5% acetyl carnitine,
- 1% *Aloe Helichrysum*,
- 3% glycerol,
- 0.01% beet extract,
- 1.2% Geogard^{®} preservative,
- 0.5% sodium ascorbate.

The beet extract (extracted from the root), known as Beta Vulgaris, is hydroglyceric.

The composition is presented preferably in the form of a cream and can be prepared, for example, in the following manner.

The wax, cetomacrogol and cetostearyl alcohol are combined and the oils are added. The water is heated to about 50°C and the Geogard®, sodium ascorbate and acetyl carnitine are added to it. Thus an aqueous phase and a lipid phase are obtained.

Under turbo-emulsification, the lipid phase is added to the aqueous phase, the remaining components being added in increasing sequence and always under turbo-emulsification. Once addition of the final components is complete, the whole is stirred slowly for at least two hours for cooling and to expel air bubbles; for the latter purpose vacuum operations can also be used.

In the composition, the individual components have specific functions: the olive oil is a softener, the St. John's wort oil promotes tissue regeneration, the wax acts as a film-forming agent, the cetostearyl alcohol acts as a lipophilic thickener, the cetomacrogol (cetomacrogol 1000 is preferred) is an emulsifier, the panthenol is an antioxidant, the water, with the olive oil, is a carrier for the active ingredients, the *Aloe* extract is soothing and reduces inflammation, the acetyl carnitine promotes tissue regeneration, the *Helichrysum* is an antioxidant and reduces inflammation, the glycerol is a co-solvent, the beet is soothing, the Geogard® is a preservative and the sodium ascorbate is antioxidant/smoothing.

Together they work synergistically, optimizing the efficacy of the phytocomplexes and of the other active ingredients.

The composition in fact takes advantage of the synergy of active phytocomplexes, amino acids and vitamins whose action against states of skin inflammation occurs through different mechanisms.

In particular, the acetyl carnitine acts mainly to promote the physiological action of tissue repair which is associated both with the antioxidant effects of vitamin C, vitamin E and pro-vitamin B5, and also with the soothing, antioxidant, inflammation-reducing and regenerating effects of panthenol and of the active phytocomplexes contained in *Aloe* extract, St. John's wort and *Helichrysum.*

Such active mix is associated with the vehicle which in this case is represented by an emulsion based on olive oil and water.

The combination of components makes it possible to simultaneously create a film-forming effect, which on the one hand can carry out a function of protecting the skin, and on the other hand can promote the permeation of the active ingredients through the horny layer, the principal skin barrier. By way of a mechanism of adjusting cutaneous transpiration, the formulation simultaneously enables a deep moisturizing of the thickened keratinocytes, with consequent increase of their permeability, and the maintenance of homeostatic skin conditions that favor the repair process.

By virtue of these capabilities, the composition lends itself to the treatment of states of skin inflammation, which can be associated with thickening and flaking, such as for example psoriasis, lichen, dermatitis and erythema.

The invention is therefore capable of creating the hydration conditions that make it possible to break through the horny layer and therefore for the active ingredients to carry out their actions.

It should be noted that the composition has a high percentage of functional ingredients with respect to the total of the components and therefore a low percentage of structural excipients.

From the results of verification tests conducted on the formulation it has been seen that the ability of vitamin E acetate (tocopherol acetate) to permeate the horny layer increases if this is added to the composition.

Operation of the composition, according to the invention, is evident from the foregoing description and, in particular, it is evident that the composition is capable of recreating the physiological conditions in the area treated by acting simultaneously to:
- create the correct degree of hydration in the area treated,
- create, in the area treated, an environment protected from bacterial proliferation and from the action of free radicals,
- reduce the typical symptoms associated with a state of inflammation (flushing, heat and pain) by acting as a soothing/astringent agent.

In practice it has been found that the invention fully achieves the intended aim and objects by providing a composition by virtue of which it is possible not only to reduce states of inflammation, but also to create the conditions of hydration that make it possible to break through the horny layer, thus optimizing the action of the active ingredients.

## Claims

1. A composition for the treatment of forms of skin inflammation, which is **characterized in that** it comprises at least the following components:
- olive oil,
- St John's wort oil,
- wax,
- cetostearyl alcohol,
- cetomacrogol,
- panthenol,
- tocopheryl acetate,
- water,
- freeze-dried *Aloe* extract,
- acetyl carnitine,
- *Helichrysum*,
- glycerol,
- a Geogard® preservative,
- sodium ascorbate.

2. The composition according to claim 1, **characterized in that** it also comprises beet extract.

3. The composition according to one or more of the preceding claims, **characterized in that** it comprises the following components referred to 100 and in the following percentages:
- 13% olive oil,
- 3% St John's wort oil,
- 1% wax,
- 4% cetostearyl alcohol,
- 1.5% cetomacrogol,
- 0.5% panthenol,
- 1% tocopheryl acetate,
- 68.79% water,
- 1% freeze-dried *Aloe* extract,
- 0.5% acetyl carnitine,
- 1% *Aloe Helichrysum*,
- 3% glycerol,
- 0.01% beet extract, Geogard® preservative,
- 1.2%
- 0.5% sodium ascorbate.

## Patentansprüche

1. Eine Zusammensetzung zur Behandlung von Hautentzündungsformen, die **dadurch gekennzeichnet ist, dass** sie mindestens die folgenden Komponenten umfasst:
- Olivenöl,
- Johanniskrautöl,
- Wachs,
- Cetostearylalkohol,
- Cetomacrogol,
- Panthenol,
- Tocopherylacetat,
- Wasser,
- gefriergetrockneten Aloe-Extrakt,
- Acetylcarnitin,
- *Helichrysum*,
- Glycerol,
- einen Geogard®-Konservierungsstoff,
- Natriumascorbat.

2. Die Zusammensetzung gemäß Anspruch 1, **dadurch gekennzeichnet, dass** sie außerdem Rübenextrakt umfasst.

3. Die Zusammensetzung gemäß einem oder mehreren der obigen Ansprüche, **dadurch gekennzeichnet, dass** sie die folgenden Komponenten, bezogen auf 100 und in folgenden Prozentsätzen, umfasst:
- 13% Olivenöl,
- 3% Johanniskrautöl,
- 1% Wachs,
- 4% Cetostearylalkohol,
- 1,5% Cetomacrogol,
- 0,5% Panthenol,
- 1% Tocopherylacetat,
- 68,79% Wasser,
- 1% gefriergetrockneten Aloe-Extrakt,
- 0,5% Acetylcarnitin,
- 1% *Helichrysum*,
- 3% Glycerol,
- 0,01% Rübenextrakt,
- 1,2% Geogard®-Konservierungsstoff,
- 0,5% Natriumascorbat.

## Revendications

1. Composition pour le traitement de formes d'inflammation de la peau, qui est **caractérisée en ce qu'**elle contient au moins les composants suivants :
- huile d'olive,
- huile d'herbe de la Saint-Jean,
- cire,
- alcool cétostéarylique,
- cétomacrogol,
- panthénol,
- acétate de tocophérol,
- eau,
- extrait d'*Aloe* lyophilisé,
- acétyl-carnitine,
- *Helichrysum*,
- glycérol,
- un conservateur Geogard®,
- ascorbate de sodium.

2. Composition conforme à la revendication 1, **caractérisée en ce qu'**elle contient aussi de l'extrait de betterave.

3. Composition conforme à l'une ou plusieurs des revendications précédentes, **caractérisée en ce qu'**elle contient les composants suivants, rapportés à 100, en les pourcentages suivants :
- 13 % d'huile d'olive,
- 3 % d'huile d'herbe de la Saint-Jean,
- 1 % de cire,
- 4 % d'alcool cétostéarylique,
- 1,5 % de cétomacrogol,
- 0,5 % de panthénol,
- 1 % d'acétate de tocophérol,
- 68,79 % d'eau,
- 1 % d'extrait d'*Aloe* lyophilisé,
- 0,5 % d'acétyl-carnitine,
- 1 % d'*Helichrysum*,
- 3 % de glycérol,
- 0,01 % d'extrait de betterave,
- 1,2 % de conservateur Geogard®,
- 0,5% d'ascorbate de sodium.
